(19) Europäisches Patentamt
European Patent Office
Office européen des brevets

(11) EP 2 121 577 B1

(12) **FASCICULE DE BREVET EUROPEEN**

(45) Date de publication et mention de la délivrance du brevet:
**31.07.2013 Bulletin 2013/31**

(21) Numéro de dépôt: **08762102.5**

(22) Date de dépôt: **15.02.2008**

(51) Int Cl.:
*C07C 227/04* (2006.01) *C07C 229/08* (2006.01)

(86) Numéro de dépôt international:
**PCT/FR2008/050254**

(87) Numéro de publication internationale:
**WO 2008/104722 (04.09.2008 Gazette 2008/36)**

(54) **PROCEDE DE SYNTHESE D'ACIDES OMEGA-AMINO-ALCANOIQUES**

VERFAHREN FÜR DIE SYNTHESE VON OMEGA-AMINO-ALKANSÄUREN

METHOD FOR THE SYNTHESIS OF OMEGA-AMINO-ALKANOIC ACIDS

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **15.02.2007 FR 0753285**

(43) Date de publication de la demande:
**25.11.2009 Bulletin 2009/48**

(73) Titulaire: **ARKEMA FRANCE**
**92700 Colombes (FR)**

(72) Inventeur: **DUBOIS, Jean-Luc**
**F-69390 Millery (FR)**

(56) Documents cités:
**WO-A-03/093215**

- **MOL J C: "APPLICATION OF OLEFIN METATHESIS IN OLEOCHEMISTRY: AN EXAMPLE OF GREEN CHEMISTRY" GREEN CHEMISTRY, ROYAL SOCIETY OF CHEMISTRY, CAMBRIDGE,, GB, vol. 4, 2002, pages 5-13, XP008068603 ISSN: 1463-9262**
- **L. COTARCA ET AL.: "Efficient and scaleable methods for omega-functionalized nonanoic acids..." ORGANIC PROCESS RESEARCH AND DEVELOPMENT, vol. 5, no. 1, 2001, pages 69-76, XP002459983 GBCAMBRIDGE**
- **F.O. AYORINDE ET AL.: "Syntheses of 12-Aminododecanoic and 11-Aminoundecanoic Acids from Vernolic Acid" JOURNAL OF THE AMERICAN OIL CHEMISTS' SOCIETY., vol. 74, no. 5, 1997, pages 531-538, XP002492147 DESPRINGER, BERLIN.**
- **W.L. KOHLHASE ET AL.: "9-Aminononanamide and Nylon-9 From Azelaaldehydic Derivatives of Soybean Oil" JOURNAL OF THE AMERICAN OIL CHEMISTS' SOCIETY., vol. 47, 1970, pages 183-188, XP002492148 DESPRINGER, BERLIN.**
- **R.L. HOLMES ET AL.: "Preparation of Dodecylamine and 6-Aminohexanoic acid from Petroselinic Acid" JOURNAL OF THE AMERICAN OIL CHEMISTS' SOCIETY., vol. 39, 1962, pages 411-414, XP002492149 DESPRINGER, BERLIN.**
- **W.R.MILLER ET AL: INDUSTRIAL AND ENGINEERING CHEMISTRY, PRODUCT RESEARCH AND DEVELOPMENT., vol. 10, no. 4, 1971, pages 442-447, XP002492150 USAMERICAN CHEMICAL SOCIETY. WASHINGTON.**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 121 577 B1

## Description

L'invention vise un procédé de synthèse d'acides ω-amino-alcanoiques ou d'esters de ces acides à partir d'acides gras naturels comportant au moins une étape de métathèse d'un acide gras mono-insaturé.

L'évolution actuelle en matière d'environnement conduit dans les domaines de l'énergie et de la chimie à privilégier l'exploitation des matières premières naturelles provenant d'une source renouvelable.

Un exemple de procédé industriel utilisant un acide gras comme matière première est celui de la fabrication à partir de l'acide ricinoléique extrait de l'huile de ricin, de l'acide amino-11-undécanoique, qui est à la base de la synthèse du Rilsan®. Ce procédé est décrit dans l'ouvrage « Les Procédés de Pétrochimie » de A. Chauvel et al. paru aux Editions TECHNIP (1986). L'acide amino-11-undécanoique est obtenu en plusieurs étapes. La première consiste en une méthanolyse de l'huile de ricin en milieu basique produisant le ricinoléate de méthyle qui est ensuite soumis à une pyrolyse pour obtenir, d'une part l'heptanaldéhyde et, d'autre part le undécylénate de méthyle. Ce dernier est passé en forme acide par hydrolyse. Ensuite l'acide formé est soumis à une hydrobromuration pour donner l'acide ω-bromé d'où l'on passe par amination à l'acide amino-11-undécanoique.

On ne trouve dans la littérature que fort peu de documents décrivant la synthèse de composés du type aminoacides à partir d'acides gras naturels. En dehors de la référence citée ci-dessus, on peut relever dans L'Encyclopedia of Chemical Technology, 4° Edition, John Wiley & Sons, (1996) Volume 19, page 501, une voie de synthèse pour la production d'un « Nylon-13 » obtenu par polymérisation d'un lactame obtenu à partir d'acide érucique produit par exemple à partir du colza. Cette voie de synthèse passe par une oxydation, par exemple par ozonolyse, pour produire un diacide à 13 atomes de carbone, l'acide brassylique. Suite à une série de transformations chimiques, le lactame 13 peut être préparé à partir de l'acide brassylique. Le lactame 13 est alors polymérisé de la même manière que le lactame 12, obtenu lui jusqu'à présent à partir de dérivés pétroliers. Ce polyamide à 13 atomes de carbone semble avoir des propriétés proches de celles des polyamides 11 et 12.

Plusieurs documents décrivent l'obtention d'aminoacides ou aminoesters d'origine renouvelable. MOL J- C. : "APPLICATION OF OLEFIN METATHESIS IN OLEOCHEMISTRY: AN EXAMPLE OF GREEN CHEMISTRY", GREEN CHEMISTRY, ROYAL SOCIETY OF CHEMISTRY, CAMBRIDGE, GB, vol. 4, 2002, pages 5-13, XP008068603 ISSN: 1463-9262, décrit un procédé de synthèse d'aminoacide consistant à soumettre le déc-9-énoate de méthyle à une reaction de métathèse catalytique croisée avec de l'éthylène. Le document de brevet WO03/093215 décrit un procédé de synthèse d'aminoacide consistant à soumettre un acide ou ester gras insaturé à une réaction de métathèse catalytique croisée avec de l'éthylène. Le document L GOTARCA ET AL: "Efficient and scaleable methods for omega-functionalized nonanoic acids...", ORGANIC PROCESS RESEARCH AND DEVELOPMENT, vol.5, no.1, 2001, pages 69-76, XP002459983 CAMBRIDGE, GB, décrit un procédé de synthèse d'acides nonanoïques oméga fonctionnalisés utiles pour la synthèse de composés de type Nylon®. Quelques documents décrivent la synthèse d'aminoacides particuliers : F.O. AYORINDE ET AL: "Syntheses of 12-Aminododecanoic and 11- Aminoundecanoic Acids from Vernolic Acid", JOURNAL OF THE AMERICAN OIL CHEMISTS' SOCIETY., vol 74, no. 5, 1997, pages 531-538, XP002492147, SPRINGER, BERLIN, DE; W.L KOHLHASE ET AL: "9-Aminononanamide and Nylon-9 From Azelaaldehydic Derivatives of Soybean Oil", JOURNAL OF THE AMERICAN OIL CHEMISTS' SOCIETY, vol. 47, 1970, pages 183-188, XP002492148, SPRINGER, BERLIN, DE; R.L. HOLMES ET AL.: "Preparation of Dodecylamine and 6-Aminohexanoic acid from Petroselinic Acid", JOURNAL OF THE AMERICAN OIL CHEMISTS' SOCIETY, vol. 39, 1962, pages 411-414, XP002492149, SPRINGER, BERLIN, DE; W.R.MILLER ET AL: INDUSTRIAL AND ENGINEERING CHEMISTRY, PRODUCT RESEARCH AND DEVELOPMENT, vol. 10, no.4,. 1971. pages 442-447, XP002492150, AMERICAN CHEMICAL SOCIETY, WASHINGTON US.

Le procédé de synthèse de l'acide amino-11-undécanoique, opéré de manière industrielle depuis plusieurs décennies donne globalement satisfaction. Cependant, il présente un certain nombre d'inconvénients. Le premier inconvénient est que sa mise en oeuvre est en pratique asservie à l'accès à une matière première spécifique, l'huile de ricin. De plus, l'huile de ricin contient une toxine : la ricine, extrêmement toxique et qu'il est nécessaire d'éliminer. Le deuxième inconvénient est lié aux réactifs utilisés, l'ammoniac et le brome en particulier, qui nécessitent des précautions de stockage et d'utilisation coûteuses. Le procédé co-produit non seulement du glycérol mais aussi de nombreux sous-produits qu'il faut valoriser séparément : de l'heptanaldéhyde, de l'estérol (mélange des esters d'acide gras non craqués).

Par ailleurs, il est important de disposer de procédés de synthèse de l'ensemble de la gamme des ω-amino-acides à chaîne longue susceptibles d'être utilisés dans l'industrie et notamment celle des polymères.

Le problème est donc de trouver un procédé de synthèse des ω-amino-acides à chaîne longue à partir de matières premières renouvelables de très large accès, et donc peu onéreuses, qui soit simple à mettre en oeuvre tout en évitant les contraintes environnementales évoquées précédemment.

La solution proposée consiste à travailler à partir des acides gras naturels à longue chaîne mono-insaturés. On entend par acide gras naturel à longue chaîne un acide issu des milieux végétal ou animal, y compris les algues, plus généralement du règne végétal, et donc renouvelable comportant au moins 10 et de préférence au moins 14 atomes de carbone par molécule.

On peut citer à titre d'exemples de tels acides, les acides en C10, les acides obtusilique (cis-4-décénoique) et caproléique (cis-9-décénoique), les acides en C12, les acides lauroléique (cis-5-dodécénoique)) et lindérique (cis-4-dodécénoique), les acides en C14, les acides myristoléique (cis-9-tétradécénoique), physétérique (cis-5-tetradécénoique) et tsuzuique (cis-4-tetradécénoique), l'acide en C16, l'acide palmitoléique (cis-9- hexadécénoique), les acides en C18, les acides oléique (cis-9-octadécénoique), élaidique (trans-9-octadécénoique), pétrosélinique (cis-6-octadécénoique), vaccénique (cis-11-octadécénoique) et ricinoléique (12-hydroxy-cis-9-octadécénoique), les acides en C20, les acides gadoléique (cis-9-eicosénoique), gondoique (cis-11-eicosénoique), l'acide cis-5-eicosènoique et l'acide lesquerolique (14-hydroxy-cis-11-eioosénoique), les acides en C22, les acides cétoléique (cis-11-docosénoique) et érucique (cis-13-docosénoique).

Ces divers acides sont issus des huiles végétales extraites de diverses plantes telles que le tournesol, le colza, le ricin, le lesquerella, l'olive, le soja, le palmier, la coriandre, le céleri, l'aneth, la carotte, le fenouil, le Limnanthes Alba (meadowfoam).

Ils sont issus également du monde animal terrestre ou marin, et dans ce dernier cas, aussi bien sous forme de poissons, de mammifères que d'algues. Il s'agit en général de graisses provenant de ruminants, de poissons comme la morue, ou de mammifères marins comme les baleines ou les dauphins.

L'invention vise un procédé de synthèse d'aminoacides ou d'aminoesters de formule générale

$$NH_2\text{-}(CH_2)_n\text{-}COOR$$

dans laquelle n représente un nombre entier compris entre 5 et 14, et R est soit H soit un radical alkyle comportant de 1 à 4 atomes de carbone, à partir d'acides ou d'esters gras naturels à longue chaîne mono-insaturés comportant au moins 10 atomes de carbone adjacents par molécule, consistant tout d'abord à transformer, dans une première étape, facultative, ledit acide ou ester gras naturel à longue chaîne, par un traitement physique, par homométathèse ou par un procédé de fermentation microbiologique, en un acide ou un ester gras mono-insaturé de formule générale

$$R_1\text{-}(CH_2)_m\text{-}CH{=}CH\text{-}(CH_2)_p\text{-}COOR$$

dans laquelle $R_1$ représente H, $CH_3$ ou un radical COOR, m un nombre entier compris entre 0 et 14 et p un nombre entier compris entre 2 et 11, puis ensuite à soumettre ledit acide ou ester gras mono-insaturé de formule générale $R_1\text{-}(CH_2)_m\text{-}CH{=}CH\text{-}(CH_2)_p\text{-}COOR$ à une réaction de métathèse catalytique croisée avec un composé de formule $R_2\text{-}CH{=}CH\text{-}R_3$ dans laquelle $R_2$ est soit H, soit CN et $R_3$ est CN ou $CH_2NH_2$, sous réserve que si $R_2$ est CN, $R_3$ ne peut être que CN, puis enfin à transformer en ω-amino-acide le produit résultant qui répond à la formule générale $R_3\text{-}CH{=}CH\text{-}(CH_2)_p\text{-}COOR$ par hydrogénation de la triple liaison de la fonction nitrile et/ou de la double liaison.

Ce procédé peut, en fonction des matières premières de départ et de l'aminoacide à synthétiser, comporter une étape préliminaire permettant de former l'acide ou ester gras mono-insaturé de formule générale $R_1\text{-}(CH_2)_m\text{-}CH{=}CH\text{-}(CH_2)_p\text{-}COOR$.

Cette première étape préliminaire facultative peut être une simple séparation physique, par tout moyen connu de l'homme de l'art permettant la purification de la charge. Elle peut être aussi une pyrolyse de l'acide/ester gras naturel permettant de parvenir à un ω-aminoacide de longueur de chaîne réduite par rapport à l'acide/ester gras naturel par réactions consécutives. Elle peut être également une réaction chimique de type homométathèse ne mettant pas en jeu de co-réactifs. Enfin elle peut être une fermentation biologique qui permet de passer sous forme diacide (diesters) les acides/esters gras naturels à longue chaîne.

Dans le procédé de l'invention, on peut traiter l'acide gras soit sous sa forme acide soit sous sa forme ester. Le passage d'une forme à l'autre, par méthanolyse, estérification ou hydrolyse, parfaitement banal ne constitue pas une transformation chimique au sens du procédé.

Le procédé de l'invention vise notamment la synthèse de l'acide amino-11-undécanoique à partir de l'acide oléique. Il consiste dans une première étape à faire réagir en présence d'un catalyseur de métathèse l'acide oléique avec l'acrylonitrile de formule $CN\text{-}CH{=}CH_2$, puis dans une deuxième étape à soumettre le produit résultant de la première étape de formule $CN\text{-}CH{=}CH\text{-}(CH_2)_7\text{-}COOH$ à une hydrogénation pour parvenir à l'acide amino-11-undécanoique.

Tous les mécanismes détaillés ci-dessous illustrent, pour faciliter l'exposé, la synthèse des acides. Cependant, la métathèse est aussi efficace avec un ester et même plus efficace, le milieu étant généralement plus anhydre. De la même manière, les schémas illustrent des réactions avec l'isomère cis des acides (ou esters) ; les mécanismes sont aussi bien applicables aux isomères trans.

Eventuellement l'ester méthylique de l'acide amino-11-undécanoique peut être polymérisé en polyamide, en dégageant du méthanol.

Le processus réactionnel est le suivant :

1) $CH_3\text{-}(CH_2)_7\text{-}CH{=}CH\text{-}(CH_2)_7\text{-}COOH + CN\text{-}CH{=}CH_2 \Leftrightarrow CN\text{-}CH{=}CH\text{-}(CH_2)_7\text{-}COOH +$

$CH_2$=CH-$(CH_2)_7$-$CH_3$

Au cours du processus la réaction ci-après peut intervenir

$CH_3$-$(CH_2)_7$-CH=CH-$(CH_2)_7$-COOH + CN-CH=$CH_2$ ⇔ CN-CH=CH-$(CH_2)_7$-$CH_3$ + $CH_2$=CH-$(CH_2)_7$-COOH

Cependant, après réaction consécutive avec l'acrylonitrile présent dans le milieu on obtiendra toujours la formation de CN-CH=CH-$(CH_2)_7$-COOH selon

$CH_2$=CH-$(CH_2)_7$-COOH + CN-CH=$CH_2$ ⇔ CN-CH=CH-$(CH_2)_7$-COOH + $CH_2$=$CH_2$

avec formation d'une oléfine comportant la fonction nitrile.

2) CN-CH=CH-$(CH_2)_7$-COOH + 3 $H_2$ → $NH_2$-$(CH_2)_{10}$-COOH.

Dans cette forme de réalisation du procédé, il est inutile de procéder à une étape préliminaire dès lors que l'acide oléique présente un degré de pureté suffisant.

Il est à noter que par ce processus on peut également obtenir l'acide amino-10 décanoique. En effet, en orientant la réaction vers le processus

$CH_3$-$(CH_2)_7$-CH=CH-$(CH_2)_7$-COOH + CN-CH=$CH_2$ ⇔ CN-CH=CH-$(CH_2)_7$-$CH_3$ + $CH_2$=CH-$(CH_2)_7$-COOH

et en travaillant en déficit d'acrylonitrile, on peut traiter l'acide 9-décénoique par hydrobromuration et amination, pour synthétiser l'acide amino-10-décanoique.

Dans une variante du procédé, on peut utiliser pour la réaction de métathèse croisée l'acide gras en C18 mis sous sa forme diacide. Dans ce cas, lors d'une étape préliminaire, l'acide oléique est transformé en diacide soit par homométathèse de l'acide oléique, soit transformé en diacide par fermentation.

Le processus réactionnel est alors le suivant :

1) HOOC-$(CH_2)_7$-CH=CH-$(CH_2)_7$-COOH + CN-CH=$CH_2$ ⇔ CN-CH=CH-$(CH_2)_7$-COOH + $CH_2$=CH-$(CH_2)_7$-COOH

et par réaction consécutive avec l'acrylonitrile :

$CH_2$=CH- $(CH_2)_7$-COOH + CN-CH=$CH_2$ ⇔ CN-CH=CH-$(CH_2)_7$-COOH + $CH_2$=$CH_2$

2) CN-CH=CH-$(CH_2)_7$-COOH + 3 $H_2$ → $NH_2$-$(CH_2)_{10}$-COOH

On peut observer, qu'au cours de la réaction, se forme l'acide 9-décénoique qui, lui aussi, si l'on travaille en excès d'acrylonitrile va entraîner la formation par métathèse croisée avec l'acrylonitrile du composé de formule CN-CH=CH-$(CH_2)_7$-COOH qui conduit également après hydrogénation à l'acide amino-11-undécanoique avec production d'éthylène. Un avantage important du procédé est ainsi l'absence de co-produit, hormis l'éthylène qui est facilement éliminé.

Le mécanisme réactionnel de cette réaction est illustrée par le schéma 1 ci-dessous

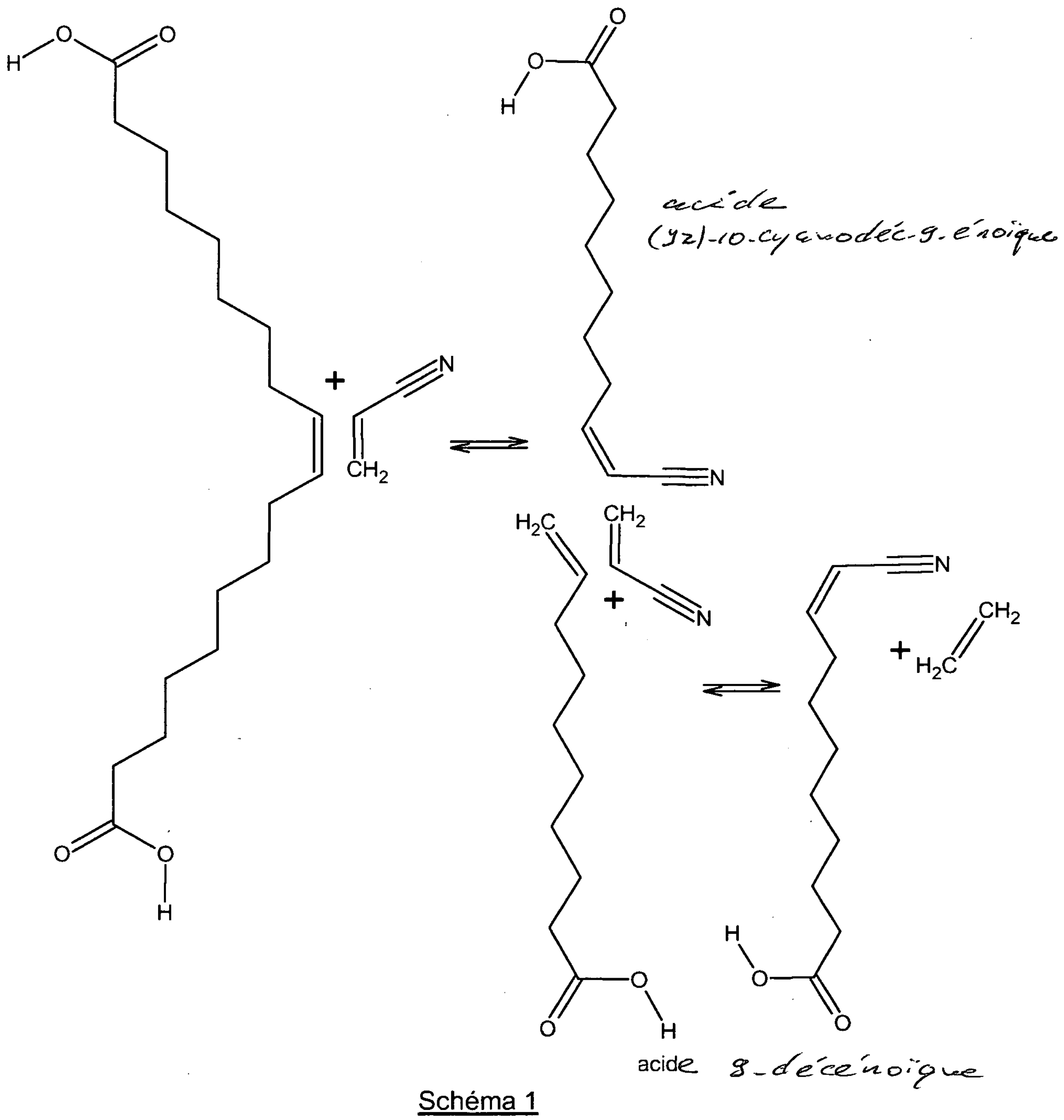

Schéma 1

Dans une autre variante du procédé utilisant comme co-réactif de métathèse croisée le dinitrile (but-2-ènedinitrile), on peut obtenir directement deux molécules $CN\text{-}CH{=}CH\text{-}(CH_2)_7\text{-}COOH$ sans co-produit, ce qui améliore notablement les performances du procédé.

Le processus réactionnel avec le but-2-ènedinitrile est alors le suivant :

$$HOOC\text{-}(CH_2)_7\text{-}CH{=}CH\text{-}(CH_2)_7\text{-}COOH + CN\text{-}CH{=}CH\text{-}CN \Leftrightarrow 2\ CN\text{-}CH{=}CH\text{-}(CH_2)_7\text{-}COOH$$

$$2 \qquad CN\text{-}CH{=}CH\text{-}(CH_2)_7\text{-}COOH + 6\ H_2 \rightarrow 2\ NH_2\text{-}(CH_2)_{10}\text{-}COOH$$

Comme on peut l'observer, le procédé de l'invention permet de synthétiser à partir d'une seule molécule d'acide en C18, du diacide en fait, deux molécules d'acide en C11 précurseur de l'acide amino-11-undécanoique, et cela quel que soit le réactif nitrile, acrylonitrile ou dinitrile utilisé. Il est clair que cette spécificité confère au procédé un avantage économique important ; elle évite en effet d'avoir à se préoccuper de la valorisation des co-produits usuels de la réaction, par exemple l'heptanal et/ou ses dérivés, dans le procédé industriel par pyrolyse de l'acide ricinoléique.

Pour la synthèse de l'acide amino-8-octanoique, on peut utiliser comme matière première acide gras naturel l'acide pétrosélinique (cis-6-octadécénoique). Dans l'hypothèse où cet acide est d'une pureté suffisante, on le soumet

à une métathèse croisée avec l'acrylonitrile et le produit issu de cette réaction est ensuite hydrogéné.

Le mécanisme réactionnel de cette réaction est le suivant.

1) $CH_3\text{-}(CH_2)_{10}\text{-}CH{=}CH\text{-}(CH_2)_4\text{-}COOH + CN\text{-}CH{=}CH_2 \Leftrightarrow CN\text{-}CH{=}CH\text{-}(CH_2)_4\text{-}COOH + CH_2{=}CH\text{-}(CH_2)_{10}\text{-}CH_3$

Au cours du processus la réaction ci-après peut intervenir

$CH_3\text{-}(CH_2)_{10}\text{-}CH{=}CH\text{-}(CH_2)_4\text{-}COOH + CN\text{-}CH{=}CH_2 \Leftrightarrow CN\text{-}CH{=}CH\text{-}(CH_2)_{10}\text{-}CH_3 + CH_2{=}CH\text{-}(CH_2)_4\text{-}COOH$

Cependant, après réaction consécutive avec l'acrylonitrile présent dans le milieu, on obtiendra toujours la formation de $CN\text{-}CH{=}CH\text{-}(CH_2)_4\text{-}COOH$ selon

$CH_2{=}CH\text{-} (CH_2)_4\text{-}COOH + CN\text{-}CH{=}CH_2 \Leftrightarrow CN\text{-}CH{=}CH\text{-}(CH_2)_4\text{-}COOH + CH_2{=}CH_2$

avec formation d'une oléfine comportant la fonction nitrile.

2) $CN\text{-}CH{=}CH\text{-}(CH_2)_4\text{-}COOH + 3\ H_2 \rightarrow NH_2\text{-}(CH_2)_7\text{-}COOH.$

Pour la synthèse de l'acide amino-7-heptanoique, on peut utiliser comme matière première acide gras naturel l'acide pétrosélinique (cis-6-octadécénoique). Dans l'hypothèse où cet acide est d'une pureté suffisante, on le soumet à une métathèse croisée avec l'éthylène (éthénolyse), puis on procède à une amination de la double liaison terminale de l'acide hepténoique intermédiaire.

Le mécanisme réactionnel de cette réaction est le suivant :

1) $CH_3\text{-}(CH_2)_{10}\text{-}CH{=}CH\text{-}(CH_2)_4\text{-}COOH + CH_2{=}CH_2 \Leftrightarrow CH_2{=}CH\text{-}(CH_2)_4\text{-}COOH + CH_2{=}CH\text{-}(CH_2)_{10}\text{-}CH_3$

2) $CH_2{=}CH\text{-}(CH_2)_4\text{-}COOH + HBr \rightarrow CH_2Br\text{-}(CH_2)_5\text{-}COOH + NH_3 \rightarrow CH_2NH_2\text{-}(CH_2)_5\text{-}COOH$

Le mécanisme réactionnel de la première étape de cette réaction est illustré par le schéma 2 ci-dessous.

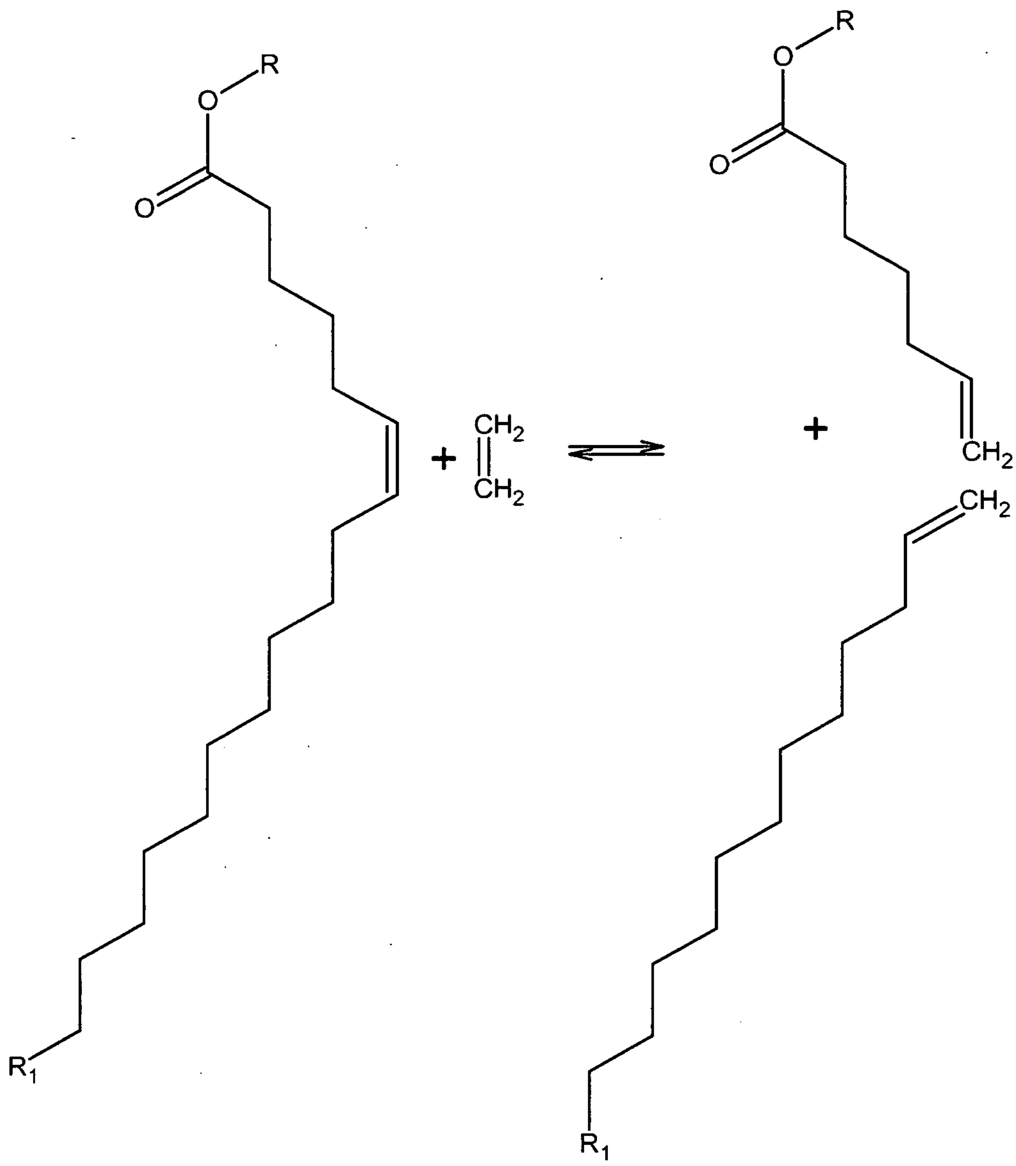

Schéma 2

On peut observer qu'il est aussi possible d'obtenir l'acide amino-8-octanoique à partir de l'acide hepténoique issu de la première étape ci-dessus en effectuant une métathèse croisée avec l'acrylonitrile.

Pour la synthèse de l'acide amino-12-dodécanoique, on peut utiliser comme matière première acide gras, l'acide ricinoléique. Cet acide, sous sa forme ester méthylique, est soumis tout d'abord à titre d'étape préliminaire à une pyrolyse thermique et la fraction acide, après hydrolyse de l'ester, issue de cette pyrolyse est soumise à une métathèse croisée avec l'acrylonitrile avant d'être hydrogénée.

Le schéma simplifié du processus réactionnel est alors le suivant.

1) $CH_3-(CH_2)_5-CHOHCH_2-CH=CH-(CH_2)_7-COOCH_3$ ($\Delta$) $\rightarrow$ $CH_3-(CH_2)_5-CHO$ + $CH_2=CH-(CH_2)_8-COOCH_3$

2) $CH_2=CH-(CH_2)_8COOH + CN-CH=CH_2 \Leftrightarrow CN-CH=CH-(CH_2)_8-COOH + CH_2=CH_2$

3) $CN-CH=CH-(CH_2)_8-COOH + 3\ H_2 \rightarrow NH_2-(CH_2)_{11}-COOH$.

Pour la synthèse de l'acide amino-12-dodécanoique à partir de l'acide ricinoléique, on peut substituer dans la réaction de métathèse croisée l'acrylonitrile par l'allyle-amine $CH_2=CH-CH_2NH_2$.

Le schéma réactionnel devient alors :

2) $CH_2=CH-(CH_2)_8COOH + CH_2=CH-CH_2NH_2 \Leftrightarrow NH_2CH_2-CH=CH-(CH_2)_8-COOH + CH_2=CH_2$

3) $NH_2CH_2-CH=CH-(CH_2)_8-COOH + H_2 \rightarrow NH_2-(CH_2)_{11}-COOH.$

Pour la synthèse de l'acide amino-14-tétradécanoique, on peut utiliser l'acide lesquérolique à titre de matière première acide gras naturel. L'acide lesquérolique, de pureté suffisante, est soumis, sous sa forme ester méthylique, tout d'abord à titre d'étape préliminaire à une pyrolyse et la fraction acide, après hydrolyse de l'ester, issue de cette pyrolyse est soumise à une métathèse croisée avec l'acétonitrile avant d'être hydrogénée.

Le processus réactionnel est le suivant.

1) $CH_3-(CH_2)_5-CHOHCH_2-CH=CH-(CH_2)_9-COOH\ (\Delta) \rightarrow CH_3-(CH_2)_5-CHO + CH_2=CH-(CH_2)_{10}COOH$

2) $CH_2=CH-(CH_2)_{10}COOH + CN-CH=CH_2 \Leftrightarrow CN-CH=CH-(CH_2)_{10}-COOH + CH_2=CH_2$

3) $CN-CH=CH-(CH_2)_{10}-COOH + 3\ H_2 \rightarrow NH_2-(CH_2)_{13}-COOH.$

Pour la synthèse de l'acide amino-15-pentadécanoique, on peut utiliser l'acide érucique à titre de matière première acide gras naturel. Ce dernier, de pureté suffisante, est soumis à une métathèse croisée avec l'acrylonitrile (ou le dinitrile) et la fraction acide est ensuite hydrogénée.

Le processus réactionnel est le suivant.

1) $CH_3-(CH_2)_7-CH=CH-(CH_2)_{11}-COOH + CN-CH=CH_2 \Leftrightarrow HOOC-(CH_2)_{11}-CH=CH-CN + CH_3-(CH_2)_7-CH=CH_2$

Au cours du processus la réaction ci-après peut intervenir

$CH_3-(CH_2)_7-CH=CH-(CH_2)_{11}-COOH + CN-CH=CH_2 \Leftrightarrow CN-CH=CH-(CH_2)_7-CH_3 + CH_2=CH-(CH_2)_{11}-COOH$

Cependant après réaction consécutive avec l'acrylonitrile présent dans le milieu, on obtiendra toujours la formation de $CN-CH=CH-(CH_2)_{11}-COOH$ selon

$CH_2=CH-(CH_2)_{11}-COOH + CN-CH=CH_2 \Leftrightarrow CN-CH=CH-(CH_2)_{11}-COOH + CH_2=CH_2$

avec formation d'une oléfine comportant la fonction nitrile.

2) $CN-CH=CH-(CH_2)_{11}-COOH + 3\ H_2 \rightarrow NH_2-(CH_2)_{14}-COOH$

On peut également envisager de passer préalablement à la métathèse croisée à une homométathèse de l'acide érucique.

Le processus réactionnel est le suivant

1) $CH_3-(CH_2)_7-CH=CH-(CH_2)_{11}-COOH + CH_3-(CH_2)_7-CH=CH-(CH_2)_{11}-COOH \Leftrightarrow HOOC-(CH_2)_{11}-CH=CH-(CH_2)_{11}-COOH + CH_3-(CH_2)_7-CH=CH-(CH_2)_7-CH_3$

2) $HOOC-(CH2)_{11}-CH=CH-(CH2)_{11}-COOH + 2\ CN-CH=CH_2 \Leftrightarrow 2\ HOOC-(CH_2)_{11}-CH=CH-CN + CH_2=CH_2$

3) $HOOC-(CH_2)_{11}-CH=CH-CN + 3H_2 \rightarrow NH_2-(CH_2)_{14}-COOH$

A partir de l'acide érucique, il est également possible d'obtenir l'acide amino-14-tétradécanoique en procédant à une première étape de métathèse croisée avec l'éthylène, qui permet d'obtenir l'acide tetra-13-décénoique, qui peut être ensuite aminé selon le processus décrit ci-dessus.

Pour la synthèse de l'acide amino-7-heptanoique, on peut utiliser comme matière première l'acide cis-5-eicosénoique.

Le mécanisme réactionnel est le suivant.

1) $CH_3-(CH_2)_{13}-CH=CH-(CH_2)_3-COOH + CN-CH=CH_2 \Leftrightarrow HOOC-(CH_2)_3-CH=CH-CN + CH_3-(CH_2)_{13}-CH=CH_2$

Au cours du processus la réaction ci-après peut intervenir

$CH_3-(CH_2)_{13}-CH=CH-(CH_2)_3-COOH + CN-CH=CH_2 \Leftrightarrow CN-CH=CH-(CH_2)_{13}-CH_3 + CH_2=CH-(CH_2)_3-COOH$

Cependant, après réaction consécutive avec l'acrylonitrile présent dans le milieu, on obtiendra toujours la formation de $CN-CH=CH-(CH_2)_3-COOH$ selon

$CH_2=CH- (CH_2)_3-COOH + CN-CH=CH_2 \Leftrightarrow CN-CH=CH-(CH_2)_3-COOH + CH_2=CH_2$

avec formation d'une oléfine comportant la fonction nitrile.

2) $CN-CH=CH-(CH_2)_3-COOH + 3 H_2 \rightarrow NH_2-(CH_2)_6-COOH$

Les réactions de métathèse sont connues depuis longtemps même si leurs applications industrielles sont relativement limitées. On peut se référer à propos de leur utilisation dans la transformation des acides (esters) gras, à l'article de J.C. Mol « Catalytic metathesis of unsaturated fatty acid esters and oil » paru dans Topics in Catalysis Vol. 27, Nos. 1-4, February 2004 (Plenum Publishing Corporation).

La catalyse de la réaction de métathèse a fait l'objet de très nombreux travaux et le développement de systèmes catalytiques sophistiqués. On peut citer par exemple les complexes au tungstène développés par Schrock et al (J. Am. Chem. Soc. 108 (1986) 2771 ou Basset et al. Angew. Chem., Ed. Engl. 31 (1992) 628. Plus récemment, sont apparus les catalyseurs dits de Grubbs (Grubbs et al. Angew. Chem., Ed. Engl. 34 (1995) 2039 et Organic Lett. 1 (1999) 953) qui sont des complexes ruthénium-benzylidène. Il s'agit de catalyse homogène. Il a été aussi développé des catalyseurs hérétogènes à base de métaux tels que Rhénium, Molybdène et Tungstène déposés sur alumine ou silice. Enfin des travaux ont été conduits pour la réalisation de catalyseurs immobilisés, c'est-à-dire de catalyseurs dont le principe actif est celui du catalyseur homogène, notamment les complexes ruthénium-carbène, mais qui est immobilisé sur un support inactif. L'objectif de ces travaux est d'augmenter la sélectivité de la réaction vis-à-vis des réactions parasites telles que les « homo-métathèse » entre les réactifs mis en présence. Ils portent non seulement sur la structure des catalyseurs mais également sur l'incidence du milieu réactionnel et les additifs pouvant être introduits.

Dans le procédé de l'invention, tout catalyseur de métathèse actif et sélectif pourra être utilisé. On utilisera cependant de préférence des catalyseurs à base de ruthénium.

La réaction de métathèse de la première étape est conduite à une température comprise entre 20 et 100°C.

Au terme de la première étape, on sépare le nitrile de l'acide undécylénique du milieu par exemple par distillation pour soumettre ce dernier à une hydrogénation.

L'invention décrit en outre un ou des aminoacides ou aminoesters d'origine renouvelable obtenus selon le procédé de la revendication 1, de formule générale $NH_2-(CH_2)_n-COOR$ dans laquelle n représente un nombre entier compris entre 5 et 14 , et R est soit H, soit un radical alkyle comportant de 1 à 4 atomes de carbone.

« Par aminoacides ou aminoesters d'origine renouvelable, on entend les aminoacides ou aminoesters qui comprennent du carbone d'origine renouvelable.

En effet, à la différence des matériaux issus de matières fossiles, les matériaux composés en partie de matières premières renouvelables contiennent du $^{14}C$. Tous les échantillons de carbone tirés d'organismes vivants (animaux ou végétaux) sont en fait un mélange de 3 isotopes : $^{12}C$ (représentant ~ 98,892 %), $^{13}C$ (~ 1,108 %) et $^{14}C$ (traces: 1, $1.10^{-10}$ %). Le rapport $^{14}C$ /$^{12}C$ des tissus vivants est identique à celui de l'atmosphère. Dans l'environnement, le $^{14}C$ existe sous deux formes prépondérantes : sous forme minérale c'est-à-dire de gaz carbonique ($CO_2$) et sous forme organique c'est à dire de carbone intégré dans des molécules organiques.

Dans un organisme vivant, le rapport $^{14}C$ /$^{12}C$ est maintenu constant par le métabolisme car le carbone est continuellement échangé avec l'environnement. La proportion de 14C étant sensiblement constante dans l'atmosphère, il en est de même dans l'organisme, tant qu'il est vivant, puisqu'il absorbe ce $^{14}C$ comme il absorbe le $^{12}C$. Le rapport moyen de $^{14}C$ /$^{12}C$ est égal à $1{,}2\times10^{-12}$.

Le $^{12}C$ est stable, c'est-à-dire que le nombre d'atomes de $^{12}C$ dans un échantillon donné est constant au cours du temps. Le $^{14}C$, lui, est radioactif (chaque gramme de carbone d'être vivant contient suffisamment d'isotope $^{14}C$ pour donner 13,6 désintégrations par minute) et le nombre de tels atomes dans un échantillon décroît au cours du temps (t) selon la loi :

$$n = no \exp(-at)$$

dans laquelle:

- no est le nombre de $^{14}C$ à l'origine (à la mort de la créature, animal ou plante),
- n est le nombre d'atomes $^{14}C$ restant au bout du temps t,
- a est la constante de désintégration (ou constante radioactive); elle est reliée à la demi-vie.

La demi-vie (ou période), est la durée au bout de laquelle un nombre quelconque de noyaux radioactifs ou de particules instables d'une espèce donnée, est réduit de moitié par désintégration ; la demi-vie T1/2 est reliée à la constante de désintégration a par la formule aT1/2= In 2. La demi-vie du 14C vaut 5730 ans.

Compte tenu de la demi-vie (T1/2) du $^{14}C$, on considère que la teneur en $^{14}C$ est constante depuis l'extraction des matières premières végétales jusqu'à la fabrication de l'aminoacide ou aminoester, et même jusqu'à la fin de son utilisation.

Le demandeur considère qu'un aminoacide ou aminoester est en partie issu de matières premières renouvelables s'il contient au moins 20% en masse de C d'origine renouvelable sur la masse totale de carbone, de préférence au moins 50 % en masse de C d'origine renouvelable sur la masse totale de carbone.

Autrement dit, un aminoacide ou aminoester est issu de matières premières renouvelables s'il contient au moins $0{,}2.10^{-10}$% en masse de $^{14}C$, de préférence $0{,}6.10^{-10}$% en masse de $^{14}C$.

A l'heure actuelle, il existe au moins deux techniques différentes pour la mesure de la teneur en $^{14}C$ d'un échantillon :

- Par spectrométrie à scintillation liquide : Cette méthode consiste à compter des particules 'Bêta' issues de la désintégration du $^{14}C$. On mesure le rayonnement Bêta issu d'un échantillon de masse connue (nombre d'atomes $^{12}C$ connu) pendant un certain temps. Cette 'radioactivité' est proportionnelle au nombre d'atomes de $^{14}C$, que l'on peut ainsi déterminer. Le $^{14}C$ présent dans l'échantillon émet des rayonnements ß-, qui au contact d'un liquide scintillant (scintillateur) donnent naissance à des photons. Ces photons ont des énergies différentes (comprises entre 0 et 156 Kev) et forment ce que l'on appelle un spectre de $^{14}C$. Selon deux variantes de cette méthode, l'analyse porte soit sur le $CO_2$ préalablement produit par l'échantillon carboné dans une solution absorbante appropriée, soit sur le benzène après conversion préalable de l'échantillon carboné en benzène.
- Par spectrométrie de masse : L'échantillon est réduit en graphite ou en $CO_2$ gazeux, analysé dans un spectromètre de masse. Cette technique utilise un accélérateur et un spectromètre de masse pour séparer les ions $^{14}C$ des $^{12}C$ et donc déterminer le rapport des deux isotopes.

Toutes ces méthodes de mesure de la teneur en $^{14}C$ des matériaux sont décrites précisément dans les normes ASTM D 6866 (notamment D6866-06) et dans les normes ASTMD 7026 (notamment 7026-04). »

La méthode de mesure préférentiellement utilisée dans le cas des aminoacides ou aminoesters de l'invention est la spectrométrie de masse décrite dans la norme ASTM D6866-06 (« accelerator mass spectroscopy »).

L'invention est illustrée par les exemples non limitatifs suivants.

<u>Exemple 1</u>

On utilise le catalyseur complexe bispyridine ruthénium (8) décrit dans la publication de Chen-Xi Bai et al., Tetrahedron Letters, 46 (2005) 7225-7228. La synthèse est effectuée dans $CH_2Cl_2$, à une concentration de 0,05 M de réactif, à une température de 45°C, et pendant 12 heures. Les rendements sont déterminés par analyse chromatographique. Dans le cas présent, le réactif est le diacide $HOOC\text{-}(CH_2)_7\text{-}CH{=}CH\text{-}(CH_2)_7\text{-}COOH$, et on utilise 4 équivalents d'acrylonitrile (4 moles d'acrylonitrile par mole de diacide), et avec une concentration en catalyseur de 5 % mole. Le rendement en nitrile-acide $CN\text{-}CH{=}CH\text{-}(CH_2)_7\text{-}COOH$ est de 50 %.

<u>Exemple 2</u>

On utilise le catalyseur complexe ruthénium (3) décrit dans la publication de Stefan Randl et al, Synlett (2001) 10, 430. Ce composé est très stable et ne se décompose pas lorsqu'il est exposé à l'air ou à l'eau. La synthèse est effectuée dans $CH_2Cl_2$, à une concentration de 0,05 M de réactif, à une température de 45°C, et pendant 2 heures. Les rendements sont déterminés par analyse chromatographique. Dans le cas présent, le réactif est l'acide 10-undécénoique, et on utilise 2 équivalents d'acrylonitrile (2 moles d'acrylonitrile par mole d'acide) et avec une concentration en catalyseur

de 5 % mole. Le rendement en nitrile-acide CN-CH=CH-$(CH_2)_8$-COOH est de 67 %.

Exemple 3

On utilise le catalyseur complexe bispyridine ruthénium (8) décrit dans la publication de Chen-Xi Bai et al., Org. Biomol. Chem., (2005), 3, 4139-4142. La synthèse est effectuée dans $CH_2Cl_2$, à une concentration de 0,05 M de réactif, à une température de 45°C, et pendant 12 heures. Les rendements sont déterminés par analyse chromatographique. Dans le cas présent, le réactif est le diester $CH_3OOC$-$(CH_2)_7$-CH=CH-$(CH_2)$-$_7$-$COOCH_3$, et on utilise 2 équivalents d'acrylonitrile (3 moles d'acrylonitrile par mole de diester), et avec une concentration en catalyseur de 10 % mole par rapport au réactif. Le rendement en nitrile-ester CN-CH=CH-$(CH_2)_7$-$COOCH_3$ est de 70 %.

Exemple 4 : métathèse croisée undécénoate de méthyle/acrylonitrile

Dans un tube de Schlenk de 50 ml, on charge 100 mg de 10-undécénoate de méthyle (0,5 mmol), 53 mg d'acrylonitrile (1 mmol) et 10 ml de toluène distillé sur sodium-benzophénone. On rajoute 1,5 mg ($2,4.10^{-3}$ mmol) de catalyseur de Hoveyda-Grubbs de seconde génération ((1,3-bis(2,4,6-triméthylphényl)-2-imidazolidinylidène)dichloro (o-isopropoxyphénylméthylène)ruthénium, disponible chez Aldrich). Sous azote et sous agitation magnétique, on chauffe à 100°C et on laisse réagir 1 heure. Le mélange réactionnel est analysé par chromatographie en phase gaz (étalon dodécane). La conversion du 10-undécénoate de méthyle est de 94%. La sélectivité en 11-cyano-10-undécénoate de méthyle (mélange cis + trans) est de 100%. Le TON (turnover number) est de 188.

Exemple 5 : métathèse croisée undécénoate de méthyle/acrylonitrile avec aiout en continu de catalyseur

Dans un tube de Schlenk de 50 ml, on charge 100 mg de 10-undécénoate de méthyle (0,5 mmol), 53 mg d'acrylonitrile (1 mmol) et 8 ml de toluène distillé sur sodium-benzophénone. On chauffe à 80°C, puis sous azote et sous agitation magnétique, on ajoute, avec une seringue et un pousse-seringue, 0,3 mg ($0,5.10^{-3}$ mmol) de catalyseur de Hoveyda-Grubbs de seconde génération dissous dans 2 ml de toluène sur une période de 2h40. A la fin de l'ajout, on laisse réagir une heure à 80°C. Le mélange réactionnel est analysé par chromatographie en phase gaz (étalon dodécane). La conversion du 10-undécénoate de méthyle est de 94%. La sélectivité en 11-cyano-10-undécénoate de méthyle (mélange cis + trans) est de 100%. Le TON est de 940.

Exemple 6 : métathèse croisée diester $C_{18}$ insaturé/acrylonitrile

Dans un tube de Schlenk de 50 ml, on charge 170 mg de 9-octadécènedioate de méthyle (0,5 mmol), 106 mg d'acrylonitrile (2 mmol) et 10 ml de toluène distillé sur sodium-benzophénone. On rajoute 3 mg ($5.10^{-3}$ mmol) de catalyseur de Hoveyda-Grubbs de seconde génération. Sous azote et sous agitation magnétique, on chauffe à 100°C et on laisse réagir 1 heure. Le mélange réactionnel est analysé par chromatographie en phase gaz (étalon tétradécane). La conversion du diester unsaturé est de 95%. La sélectivité en 10-cyano-9-décénoate de méthyle (mélange cis + trans) est de 93% (avec 7% de sélectivité en 9-décénoate de méthyle). Le TON est de 95.

Exemple 7 : métathèse croisée diester $C_{18}$ insaturé/acrylonitrile avec ajout en continu de catalyseur

Dans un tube de Schlenk de 50 ml, on charge 170 mg de 9-octadécènedioate de méthyle (0,5 mmol), 106 mg d'acrylonitrile (2 mmol) et 8 ml de toluène distillé sur sodium-benzophénone. On chauffe à 80°C, puis sous azote et sous agitation magnétique, on ajoute, avec une seringue et un pousse-seringue, 0,6 mg ($10^{-3}$ mmol) de catalyseur de Hoveyda-Grubbs de seconde génération dissous dans 2 ml de toluène sur une période de 4h. A la fin de l'ajout, on laisse réagir une heure à 80°C. Le mélange réactionnel est analysé par chromatographie en phase gaz (étalon tétradécane). La conversion du diester insaturé est de 98%. La sélectivité en 10-cyano-9-décénoate de méthyle (mélange cis + trans) est de 96% (avec 4% de sélectivité en 9-décénoate de méthyle). Le TON est de 490.

**Revendications**

1. Procédé de synthèse d'aminoacides ou d'aminoesters de formule générale $NH_2$-$(CH_2)_n$-COOR dans laquelle n représente un nombre entier compris entre 5 et 14 , et R est soit H, soit un radical alkyle comportant de 1 à 4 atomes de carbone, à partir d'acides ou d'esters gras naturels à longue chaîne mono-insaturés comportant au moins 10 atomes de carbone adjacents par molécule, consistant tout d'abord à transformer, dans une première étape, facultative, ledit acide ou ester gras naturel à longue chaîne, par un traitement physique, par homométathèse, par

éthénolyse, ou par un procédé microbiologique, en un acide ou un ester gras mono-insaturé de formule générale $R_1$-$(CH_2)_m$-CH=CH-$(CH_2)_p$-COOR dans laquelle $R_1$ représente H, $CH_3$ ou un radical COOR, m un nombre entier compris entre 0 et 14 et p un nombre entier compris entre 2 et 11, puis ensuite à soumettre ledit acide ou ester gras mono-insaturé de formule générale $R_1(CH_2)_m$-CH=CH-$(CH_2)_p$-COOR à une réaction de métathèse catalytique croisée avec un composé de formule $R_2$-CH=CH-$R_3$ dans laquelle $R_2$ est soit H, soit CN et $R_3$ est CN ou $CH_2NH_2$, sous réserve que si $R_2$ est CN, $R_3$ ne peut être que CN, puis enfin à transformer en w-amino-acide le produit résultant qui répond à la formule générale $R_3$-CH=CH-$(CH_2)_p$-COOR par hydrogénation.

2. Procédé selon la revendication 1 **caractérisé en ce que** l'acide ou ester gras mono-insaturé synthétisé de formule générale $R_1$-$(CH_2)_m$-CH=CH-$(CH_2)_p$-COOR est soumis à une réaction de métathèse avec l'acrylonitrile.

3. Procédé selon la revendication 2 **caractérisé en ce que** l'aminoacide de formule $NH_2$-$(CH_2)_{10}$-COOH, est synthétisé par réaction de l'acrylonitrile sur l'acide oléique suivie d'une hydrogénation du composé intermédiaire de formule CN-CH=CH-$(CH_2)_7$-COOH.

4. Procédé selon la revendication 3 **caractérisé en ce que** l'aminoacide de formule $NH_2$-$(CH_2)_{10}$-COOH, est synthétisé par réaction d'un excès d'acrylonitrile sur un diacide oléique de formule HOOC-$(CH_2)_7$-CH=CH-$(CH_2)_7$-COOH suivie d'une hydrogénation du composé intermédiaire de formule CN-CH=CH-$(CH_2)_7$-COOH.

5. Procédé selon la revendication 1 **caractérisé en ce que** l'aminoacide de formule $NH_2$-$(CH_2)_{10}$-COOH, est synthétisé par réaction du but-2-ènedinitrile de formule CN-CH=CH-CN sur un diacide oléique de formule HOOC-$(CH_2)_7$-CH=CH-$(CH_2)_7$-COOH suivie d'une hydrogénation du composé intermédiaire de formule CN-CH=CH-$(CH_2)_7$-COOH.

6. Procédé selon la revendication 2 **caractérisé en ce que** l'aminoacide de formule $NH_2$-$(CH_2)_7$-COOH est préparé à partir de l'acide pétrosélinique qui est soumis à une métathèse avec l'acrylonitrile, l'acide intermédiaire de formule CN-CH=CH-$(CH_2)_4$-COOH étant ensuite hydrogéné.

7. Procédé selon la revendication 2 **caractérisé en ce que** l'aminoacide de formule $NH_2$-$(CH_2)_{11}$-COOH est préparé à partir de l'acide de formule $CH_2$=CH-$(CH_2)_8$COOH issu, lors d'une première étape, de la pyrolyse de l'acide ricinoléique, par métathèse à l'acrylonitrile, puis soumis à une hydrogénation.

8. Procédé selon la revendication 2 **caractérisé en ce que** l'aminoacide de formule $NH_2$-$(CH_2)_{13}$-COOH est préparé à partir de l'acide de formule $CH_2$=CH-$(CH_2)_{13}$COOH issu, lors d'une première étape de la pyrolyse de l'acide lesquérolique par métathèse à l'acrylonitrile, puis soumis à une hydrogénation.

9. Procédé selon la revendication 2 **caractérisé en ce que** l'aminoacide de formule $NH_2$-$(CH_2)_6$-COOH est préparé à partir de l'acide cis-5-eicosénoique par métathèse avec l'acrylonitrile puis hydrogénation.

10. Procédé selon la revendication 2 **caractérisé en ce que** l'aminoacide de formule $NH_2$-$(CH_2)_{14}$-COOH est préparé à partir de l'acide érucique par métathèse avec l'acrylonitrile puis hydrogénation.

11. Procédé selon la revendication 1 **caractérisé en ce que** l'aminoacide de formule $NH_2$-$(CH_2)_{11}$-COOH est préparé à partir de l'acide de formule $CH_2$=CH-$(CH_2)_8$COOH issu, lors d'une première étape, de la pyrolyse de l'acide ricinoléique, par métathèse à l'allyle amine, puis soumis à une hydrogénation.

**Patentansprüche**

1. Verfahren zur Synthese von Aminosäuren oder Aminoestern der allgemeinen Formel $NH_2$-$(CH_2)_n$-COOR, worin n für eine ganze Zahl zwischen 5 und 14 steht und R für H oder einen Alkylrest mit 1 bis 4 Kohlenstoffatomen steht, aus einfach ungesättigten langkettigen natürlichen Fettsäuren oder Fettestern mit mindestens 10 benachbarten Kohlenstoffatomen pro Molekül, bei dem man zunächst in einem ersten fakultativen Schritt die langkettige natürliche Fettsäure bzw. den langkettigen natürlichen Fettester durch eine physikalische Behandlung, durch Homometathese, durch Ethenolyse oder durch ein mikrobiologisches Verfahren in eine einfach ungesättigte Fettsäure bzw. einen einfach ungesättigten Fettester der allgemeinen Formel $R_1$-$(CH_2)_m$-CH=CH-$(CH_2)_p$-COOR, worin $R_1$ für H, $CH_3$ oder einen COOR-Rest steht, m für eine ganze Zahl zwischen 0 und 14 steht und p für eine ganze Zahl zwischen 2 und 11 steht, umwandelt, dann die einfach ungesättigte Fettsäure bzw. den einfach ungesättigten Fettester der

allgemeinen Formel $R_1$-$(CH_2)_m$-CH=CH-$(CH_2)_p$-COOR einer katalytischen Kreuzmetathesereaktion mit einer Verbindung der Formel $R_2$-CH=CH-$R_3$, worin $R_2$ für H oder CN und $R_3$ für CN oder $CH_2NH_2$ steht, mit der Maßgabe, dass dann, wenn $R_2$ für CN steht, $R_3$ nur für CN stehen kann, unterwirft und schließlich das resultierende Produkt, das der allgemeinen Formel $R_3$-CH=CH-$(CH_2)_p$-COOR entspricht, durch Hydrierung in eine ω-Aminosäure umwandelt.

**2.** Verfahren nach Anspruch 1, **dadurch gekennzeichnet, dass** man die synthetisierte einfach ungesättigte Fettsäure bzw. den synthetisierten einfach ungesättigten Fettester der allgemeinen Formel $R_1$-$(CH_2)_m$-CH=CH-$(CH_2)_p$-COOR einer Metathesereaktion mit Acrylnitril unterwirft.

**3.** Verfahren nach Anspruch 2, **dadurch gekennzeichnet, dass** man durch Umsetzung von Acrylnitril mit Oleinsäure und anschließende Hydrierung der intermediären Verbindung der Formel CN-CH=CH-$(CH_2)_7$-COOH die Aminosäure der Formel $NH_2$-$(CH_2)_{10}$-COOH synthetisiert.

**4.** Verfahren nach Anspruch 3, **dadurch gekennzeichnet, dass** man durch Umsetzung eines Überschusses von Acrylnitril mit einer Oleindisäure der Formel HOOC- $(CH_2)_7$-CH=CH-$(CH_2)_7$-COOH und anschließende Hydrierung der intermediären Verbindung der Formel CN-CH=CH-$(CH_2)_7$-COOH die Aminosäure der Formel $NH_2$-$(CH_2)_{10}$-COOH synthetisiert.

**5.** Verfahren nach Anspruch 1, **dadurch gekennzeichnet, dass** man durch Umsetzung von But-2-endinitril der Formel CN-CH=CH-CN mit einer Oleindisäure der Formel HOOC-$(CH_2)_7$-CH=CH-$(CH_2)_7$-COOH und anschließende Hydrierung der intermediären Verbindung der Formel CN-CH=CH-$(CH_2)_7$-COOH die Aminosäure der Formel $NH_2$-$(CH_2)_{10}$-COOH synthetisiert.

**6.** Verfahren nach Anspruch 2, **dadurch gekennzeichnet, dass** man die Aminosäure der Formel $NH_2$-$(CH_2)_7$-COOH aus Petroselinsäure herstellt, wobei man die Petroselinsäure einer Metathese mit Acrylnitril unterwirft und die intermediäre Säure der Formel CN-CH=CH-$(CH_2)_4$-COOH dann hydriert.

**7.** Verfahren nach Anspruch 2, **dadurch gekennzeichnet, dass** man die Aminosäure der Formel $NH_2$-$(CH_2)_{11}$-COOH aus der in einem ersten Schritt aus der Pyrolyse von Ricinolsäure erhaltenen Säure der Formel $CH_2$=CH-$(CH_2)_8$COOH durch Metathese mit Acrylnitril und anschließende Hydrierung herstellt.

**8.** Verfahren nach Anspruch 2, **dadurch gekennzeichnet, dass** man die Aminosäure der Formel $NH_2$-$(CH_2)_{13}$-COOH aus der in einem ersten Schritt aus der Pyrolyse von Lesquerolsäure erhaltenen Säure der Formel $CH_2$=CH-$(CH_2)_{13}$COOH durch Metathese mit Acrylnitril und anschließende Hydrierung herstellt.

**9.** Verfahren nach Anspruch 2, **dadurch gekennzeichnet, dass** man die Aminosäure der Formel $NH_2$-$(CH_2)_6$-COOH aus cis-5-Eicosensäure durch Metathese mit Acrylnitril und anschließende Hydrierung herstellt.

**10.** Verfahren nach Anspruch 2, **dadurch gekennzeichnet, dass** man die Aminosäure der Formel $NH_2$-$(CH_2)_{14}$-COOH aus Erucasäure durch Metathese mit Acrylnitril und anschließende Hydrierung herstellt.

**11.** Verfahren nach Anspruch 1, **dadurch gekennzeichnet, dass** man die Aminosäure der Formel $NH_2$-$(CH_2)_{11}$-COOH aus der in einem ersten Schritt aus der Pyrolyse von Ricinolsäure erhaltenen Säure der Formel $CH_2$=CH-$(CH_2)_8$COOH durch Metathese mit Allylamin und anschließende Hydrierung herstellt.

## Claims

**1.** A process for synthesizing amino acids or amino esters of general formula $NH_2$-$(CH_2)_n$-COOR in which n represents an integer between 5 and 14, and R is either H or an alkyl radical containing from 1 to 4 carbon atoms, from monounsaturated long-chain natural fatty acids or esters containing at least 10 adjacent carbon atoms per molecule, which consists first in converting, in an optional first step, said long-chain natural fatty acid or ester, via a physical treatment, via homometathesis, via ethenolysis, or via a microbiological process, into a monounsaturated fatty acid or ester of general formula $R_1$-$(CH_2)_m$-CH=CH-$(CH_2)_p$-COOR in which $R_1$ represents H, $CH_3$ or a radical COOR, m is an integer between 0 and 14 and p is an integer between 2 and 11, and then in subjecting said monounsaturated fatty acid or ester of general formula $R_1$-$(CH_2)_m$-CH=CH-$(CH_2)_p$-COOR to a catalytic cross-metathesis reaction with a compound of formula $R_2$-CH=CH-$R_3$ in which $R_2$ is either H or CN and $R_3$ is CN or $CH_2NH_2$, with the proviso that

if $R_2$ is CN, $R_3$ can be only CN, and then finally in converting into an ω-amino acid the resulting product corresponding to the general formula $R_3$-CH=CH-$(CH_2)_p$-COOR by hydrogenation.

2. The process as claimed in claim 1, **characterized in that** the synthesized monounsaturated fatty acid or ester of general formula $R_1$-$(CH_2)_m$-CH=CH-$(CH_2)_p$-COOR is subjected to a metathesis reaction with acrylonitrile.

3. The process as claimed in claim 2, **characterized in that** the amino acid of formula $NH_2$-$(CH_2)_{10}$-COOH is synthesized by reacting acrylonitrile with oleic acid, followed by a hydrogenation of the intermediate compound of formula CN-CH=CH-$(CH_2)_7$-COOH.

4. The process as claimed in claim 3, **characterized in that** the amino acid of formula $NH_2$-$(CH_2)_{10}$-COOH is synthesized by reacting an excess of acrylonitrile with an oleic diacid of formula HOOC-$(CH_2)_7$-CH=CH-$(CH_2)_7$-COOH, followed by a hydrogenation of the intermediate compound of formula CN-CH=CH-$(CH_2)_7$-COOH.

5. The process as claimed in claim 1, **characterized in that** the amino acid of formula $NH_2$-$(CH_2)_{10}$-COOH is synthesized by reacting 2-butenedinitrile of formula CN-CH=CH-CN with an oleic diacid of formula HOOC-$(CH_2)_7$-CH=CH-$(CH_2)_7$-COOH, followed by a hydrogenation of the intermediate compound of formula CN-CH=CH-$(CH_2)_7$-COOH.

6. The process as claimed in claim 2, **characterized in that** the amino acid of formula $NH_2$-$(CH_2)_7$-COOH is prepared from petroselinic acid, which is subjected to metathesis with acrylonitrile, the intermediate acid of formula CN-CH=CH-$(CH_2)_4$-COOH then being hydrogenated.

7. The process as claimed in claim 2, **characterized in that** the amino acid of formula $NH_2$-$(CH_2)_{11}$-COOH is prepared from the acid of formula $CH_2$=CH-$(CH_2)_8$COOH, which is obtained, in a first step, from the pyrolysis of ricinoleic acid, via metathesis with acrylonitrile, and is then subjected to a hydrogenation.

8. The process as claimed in claim 2, **characterized in that** the amino acid of formula $NH_2$-$(CH_2)_{13}$-COOH is prepared from the acid of formula $CH_2$=CH-$(CH_2)_{13}$COOH, which is obtained, in a first step, from pyrolysis of lesquerolic acid, via metathesis with acrylonitrile, and is then subjected to a hydrogenation.

9. The process as claimed in claim 2, **characterized in that** the amino acid of formula $NH_2$-$(CH_2)_6$-COOH is prepared from cis-5-eicosenoic acid via metathesis with acrylonitrile, followed by hydrogenation.

10. The process as claimed in claim 2, **characterized in that** the amino acid of formula $NH_2$-$(CH_2)_{14}$-COOH is prepared from erucic acid via metathesis with acrylonitrile, followed by hydrogenation.

11. The process as claimed in claim 1, **characterized in that** the amino acid of formula $NH_2$-$(CH_2)_{11}$-COOH is prepared from the acid of formula $CH_2$=CH-$(CH_2)_8$COOH, which is obtained, during a first step, from pyrolysis of ricinoleic acid, via metathesis with allylamine, and is then subjected to a hydrogenation.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 03093215 A

### Littérature non-brevet citée dans la description


- **A. CHAUVEL et al.** Les Procédés de Pétrochimie. 1986
- L'Encyclopedia of Chemical Technology. John Wiley & Sons, 1996, vol. 19, 501
- APPLICATION OF OLEFIN METATHESIS IN OLEOCHEMISTRY: AN EXAMPLE OF GREEN CHEMISTRY. **MOL J- C.** GREEN CHEMISTRY. ROYAL SOCIETY OF CHEMISTRY, 2002, vol. 4, 5-13
- **L GOTARCA et al.** Efficient and scaleable methods for omega-functionalized nonanoic acids... *ORGANIC PROCESS RESEARCH AND DEVELOPMENT,* vol. 5 (1), 69-76
- Syntheses of 12-Aminododecanoic and 11- Aminoundecanoic Acids from Vernolic Acid. **F.O. AYORINDE et al.** JOURNAL OF THE AMERICAN OIL CHEMISTS' SOCIETY. SPRINGER, 1997, vol. 74, 531-538
- 9-Aminononanamide and Nylon-9 From Azelaaldehydic Derivatives of Soybean Oil. **W.L KOHLHASE et al.** JOURNAL OF THE AMERICAN OIL CHEMISTS' SOCIETY. SPRINGER, 1970, vol. 47, 183-188
- Preparation of Dodecylamine and 6-Aminohexanoic acid from Petroselinic Acid. **R.L. HOLMES et al.** JOURNAL OF THE AMERICAN OIL CHEMISTS' SOCIETY. SPRINGER, 1962, vol. 39, 411-414
- **W.R.MILLER et al.** INDUSTRIAL AND ENGINEERING CHEMISTRY, PRODUCT RESEARCH AND DEVELOPMENT. AMERICAN CHEMICAL SOCIETY, 1971, vol. 10, 442-447
- Catalytic metathesis of unsaturated fatty acid esters and oil. **J.C. MOL.** Topics in Catalysis. Plenum Publishing Corporation, Février 2004, vol. 27
- **SCHROCK et al.** *J. Am. Chem. Soc.,* 1986, vol. 108, 2771
- **BASSET et al.** *Angew. Chem., Ed. Engl.,* 1992, vol. 31, 628
- **GRUBBS et al.** *Angew. Chem., Ed. Engl.,* 1995, vol. 34, 2039
- *Organic Lett.,* 1999, vol. 1, 953
- **CHEN-XI BAI et al.** *Tetrahedron Letters,* 2005, vol. 46, 7225-7228
- **STEFAN RANDL et al.** *Synlett,* 2001, vol. 10, 430
- **CHEN-XI BAI et al.** *Org. Biomol. Chem.,* 2005, vol. 3, 4139-4142